# EUROPEAN PATENT APPLICATION

(11) **EP 3 714 921 A1**
(43) Date of publication of application: **30.09.2020**
(21) Application number: 20175795.2
(22) Date of filing: 26.01.2016
(51) Int. Cl.: A61M 5/14, A61M 5/168

(54) **AIR STOP MEMBRANE FOR MAINTAINING A FLUID COLUMN IN AN IV SET**

(30) Priority: 27.01.2015 US 201562108413 P; 25.01.2016 US 201615005779
(62) Divisional of application: 16704114.4
(71) Applicant: Becton, Dickinson and Company, Franklin Lakes, NJ 07417-1880 (US)
(72) Inventor: Whitaker, Weston O., Glenmore, PA 19343 (US); Staley, Shaun, Sandy, UT 84093 (US); Munoz, Marcelino, Riverton, UT 84065 (US); Larsen, Jon, Riverton, UT 84065 (US)
(74) Representative: dompatent von Kreisler Selting Werner - Partnerschaft von Patent- und Rechtsanwälten mbB

(57) **Abstract**

An air stop membrane can be used within an IV set to maintain a fluid column within the IV set downstream of the membrane even after a fluid bag has emptied. By maintaining a fluid column downstream of the membrane, air is prevented from entering into the tubing that couples the IV set to a vascular access device. For this reason, once a fluid bag has emptied, a new fluid bag can be coupled to the IV set without needing to re-prime the IV set. Therefore, a clinician need not be present as a fluid bag is emptying to ensure that air does not enter the tubing.

## Description

### BACKGROUND

The present invention is generally directed to tubing sets used in the administration of fluids to a patient that are commonly referred to as intravascular ("IV") sets. More particularly, the present invention is directed to an air stop membrane that can be included within an IV set to maintain a fluid column in the IV set even after a fluid bag has emptied. An IV set according to the invention is used broadly herein to describe tubing sets used in the arterial, intravenous, intravascular, peritoneal, and non-vascular administration of fluid. Of course, one of skill in the art may use an IV set to administer fluids to other locations within a patient's body.

One common method of administering fluids into a patient's blood flow is through an IV set. An IV set is an apparatus that generally includes a connector for connection to a fluid bag, a drip chamber used to determine the flow rate of fluid from the fluid bag, tubing for providing a connection between the fluid bag and the patient, and a connector for attachment to a catheter that may be positioned intravenously in a patient. An IV set may also include a Y-connector that allows for the piggybacking of IV sets and for the administration of medicine from a syringe into the tubing of the IV set.

It is a generally good practice to remove air from IV sets which access a patient's blood flow. While this concern is critical when accessing arterial blood, it is also a concern when accessing the venous side. Specifically, if air bubbles are allowed to enter a patient's blood stream while receiving the intravenous administration of fluids, the air bubbles can form an air embolism and cause serious injury to a patient.

Normally, in a majority of adults, the right atrium and the left atrium are completely separated from each other so that the blood and air bubbles are moved from the right atrium, to the right ventricle, and then to the lungs where the air bubbles may be safely vented. The bubble-free blood is then returned to the left atrium, where the blood is moved to the left ventricle and then sent throughout the body.

However, in infants and in a small portion of the adult population, the right atrium and left atrium are not completely separated. Consequently, air bubbles can move directly from the right atrium into the left atrium and then be dispersed throughout the body. As a result, these air bubbles may cause strokes, tissue damage, and/or death. Therefore, it is important to prevent air bubbles from entering a patient's blood stream.

In spite of the importance of removing air bubbles while priming an IV set for use in the intravenous administration of fluids, the complete removal of air bubbles can be a time consuming process. The process may also lead to contamination of the IV set by inadvertently touching a sterile end of the IV set. Typically, when an IV set is primed, a clamp is closed to prevent fluid from moving from a drip chamber through the tubing. The IV set is then attached to an IV bag or bottle. Once attached, the drip chamber, which is typically made of a clear flexible plastic, may be squeezed to draw the fluid out of the IV bag or bottle and into the drip chamber. The drip chamber is allowed to fill about ⅓ to ½ full when the clamp is opened to allow fluid to flow through the tube to an end of the IV set.

This initial process, however, typically traps air in tubing which must be removed. For example, the flow of the fluid through the tubing of the IV set may be turbulent and can entrap air within the tube as the boundary layer between the fluid and the tubing is sheared. The flow rate out of the drip chamber may be higher than the flow rate of fluid entering the drip chamber. This can cause a bubble ladder to form as air is sucked from the drip chamber into the tubing.

Additionally, air bubbles may be generated as drops of fluid strike the surface of the pool of fluid within the drip chamber. These air bubbles can be pulled into the tubing of the IV set from the drip chamber. This problem may be aggravated in pediatric applications where the drip orifice may be smaller which may result in increased turbulence.

To remove air bubbles from the IV set, fluid from the IV bag or bottle is allowed to flow through the tubing while an attendant taps the tubing to encourage the air bubbles out the end of the IV set. As the fluid is allowed to flow out of the IV set to clear air bubbles from the tubing, the fluid is generally allowed to flow into a waste basket or other receptacle. During this procedure the end of the tubing may contact the waste basket or be touched by the attendant and thus, become contaminated. An additional shortcoming of this debubbling process is that it requires attention and time that could have been used to perform other tasks that may be valuable to the patient.

Another debubbling method is to directly remove air bubbles from the IV set. More specifically, if the IV set includes a Y-connector, air bubbles may be removed at the Y-connector by a syringe.

To address the difficulties of removing bubbles from an IV set, various prior art IV set designs have employed a membrane for filtering air from the fluid as it flows through the IV set. For example, oftentimes a membrane may be placed in the bottom of the drip chamber so that fluid flowing out of the drip chamber must pass through the membrane. The membrane can be configured to allow the passage of fluid while blocking the passage of air. In this way, bubbles are prevented from passing into the tubing leading to the patient. Similarly, a membrane can be included in the connector that couples the tubing to a catheter to block any air present in the tubing from passing into the patient's vasculature.

The use of air filtering membranes in these prior art IV set designs have been beneficial. However, even with the use of these membranes, various drawbacks still exist. For example, if a fluid bag is allowed to empty, all of the fluid within the IV set will pass through the IV set and into the patient leaving the IV set full of air. Once this occurs, the IV set will have to be re-primed to remove the air from the IV set before a new fluid bag can be administered. To avoid having to re-prime the IV set, clinicians will therefore have to be present as a fluid bag is emptying to ensure that the fluid bag can be replaced before the drip chamber empties.

Also, if the clinician does not notice that air has entered into the tubing, he or she may fail to re-prime the IV set when connecting a new fluid bag. This may result in air passing into the patient once the new fluid bag is administered.

### BRIEF SUMMARY OF THE INVENTION

Embodiments of the present invention are generally directed to an air stop membrane that can be used within an IV set to maintain a fluid column within the IV set downstream of the membrane even after a fluid bag has emptied. By maintaining a fluid column downstream of the membrane, air is prevented from entering into the tubing that couples the IV set to the patient (e.g., via a catheter). For this reason, once a fluid bag has emptied, a new fluid bag can be coupled to the IV set without needing to re-prime the IV set. Therefore, when using an IV set in accordance with embodiments of the present invention, a clinician need not be present as a fluid bag is emptying to ensure that air does not enter the tubing.

A membrane in accordance with embodiments of the present invention can be configured to have a bubble point pressure that is sufficient to block the passage of air through the membrane once the membrane is exposed to air after the administration of a fluid bag. Because the membrane blocks the passage of air, the membrane forms a seal to maintain the negative pressure within the IV set downstream of the membrane. By maintaining this pressure, the fluid downstream of the membrane will remain within the IV set due to capillary action within the air stop membrane.

The membrane can be positioned in different locations within an IV set including, for example, within the drip chamber, within the spike, or within a separate component positioned either below or above the drip chamber. The membrane can be formed of a hydrophilic porous material having a pore size and cross-sectional area selected to provide an adequate flow rate while also providing a sufficient bubble point pressure to maintain a fluid column. The optimal pore size and cross-sectional area of a membrane can vary based on the location of the membrane within the IV set.

In one embodiment, the present invention is implemented as an IV set that includes a drip chamber; a spike for fluidly coupling the drip chamber to a fluid bag; tubing for coupling the drip chamber to a vascular access device; and an air stop membrane that maintains a fluid column within the IV set below the air stop membrane after a fluid level within the IV set reaches the air stop membrane.

In another embodiment, the present invention is implemented as a membrane housing that includes an enclosed body having an input port and an output port; and an air stop membrane positioned within the enclosed body to separate the input port from the output port. The air stop membrane supports a fluid column below the air stop membrane after a fluid level within the enclosed body reaches the air stop membrane. In some embodiments, the membrane housing can include an air vent to allow for re-priming of an IV set in which the membrane housing is used.

In another embodiment, the present invention is implemented as an IV set that includes a drip chamber; a spike for fluidly coupling the drip chamber to a fluid bag; tubing for coupling the drip chamber to a vascular access device; and an air stop membrane positioned within the drip chamber or spike and comprising a hydrophilic material having a bubble point pressure of approximately 5.0 psig to cause a fluid column to be maintained within the IV set below the air stop membrane after a fluid level within the IV set reaches the air stop membrane.

These and other features and advantages of the present invention may be incorporated into certain embodiments of the invention and will become more fully apparent from the following description and appended claims, or may be learned by the practice of the invention as set forth hereinafter. The present invention does not require that all the advantageous features and all the advantages described herein be incorporated into every embodiment of the invention.

### BRIEF DESCRIPTION OF THE SEVERAL VIEWS OF THE DRAWINGS

In order that the manner in which the above-recited and other features and advantages of the invention are obtained will be readily understood, a more particular description of the invention briefly described above will be rendered by reference to specific embodiments thereof that are illustrated in the appended drawings. These drawings depict only typical embodiments of the invention and are not therefore to be considered to limit the scope of the invention.
Figure 1 is a perspective view of a first embodiment of an IV set that includes an air stop membrane within the drip chamber;
Figure 1A is a cross-sectional view of the IV set of Figure 1 when the IV set is coupled to a fluid bag that contains fluid;
Figure 1B is a cross-sectional view of the IV set of Figure 1 when the IV set is coupled to a fluid bag that has emptied;
Figure 2 is a perspective view of a second embodiment of an IV set that includes an air stop membrane within the spike;
Figure 2A is a cross-sectional view of the IV set of Figure 2 when the IV set is coupled to a fluid bag that contains fluid;
Figure 2B is a cross-sectional view of the IV set of Figure 2 when the IV set is coupled to a fluid bag that has emptied;
Figure 3 is a perspective view of a third embodiment of an IV set that includes a separate membrane housing that includes an air stop membrane;
Figure 3A is a cross-sectional view of the IV set of Figure 3 when the IV set is coupled to a fluid bag that contains fluid;
Figure 3B is a cross-sectional view of the IV set of Figure 3 when the IV set is coupled to a fluid bag that has emptied; and
Figure 4 is a cross-sectional view of the IV set of Figure 3 that includes an alternate embodiment of a separate membrane housing.

### DETAILED DESCRIPTION OF THE INVENTION

The presently preferred embodiments of the present invention can be understood by reference to the drawings, wherein like reference numbers indicate identical or functionally similar elements. It will be readily understood that the components of the present invention, as generally described and illustrated in the figures herein, could be arranged and designed in a wide variety of different configurations. Thus, the following more detailed description, as represented in the figures, is not intended to limit the scope of the invention as claimed, but is merely representative of presently preferred embodiments of the invention.

Moreover, the Figures may show simplified or partial views, and the dimensions of elements in the Figures may be exaggerated or otherwise not in proportion for clarity. In addition, the singular forms "a," "an," and "the" include plural referents unless the context clearly dictates otherwise. Thus, for example, reference to a terminal includes reference to one or more terminals. In addition, where reference is made to a list of elements (e.g., elements a, b, c), such reference is intended to include any one of the listed elements by itself, any combination of less than all of the listed elements, and/or a combination of all of the listed elements.

The term "substantially" means that the recited characteristic, parameter, or value need not be achieved exactly, but that deviations or variations, including for example, tolerances, measurement error, measurement accuracy limitations and other factors known to those of skill in the art, may occur in amounts that do not preclude the effect the characteristic was intended to provide.

As used herein, the term "proximal", "top", "up" or "upwardly" refers to a location on the device that is closest to the clinician using the device and farthest from the patient in connection with whom the device is used when the device is used in its normal operation. Conversely, the term "distal", "bottom", "down" or "downwardly" refers to a location on the device that is farthest from the clinician using the device and closest to the patient in connection with whom the device is used when the device is used in its normal operation.

As used herein, the term "in" or "inwardly" refers to a location with respect to the device that, during normal use, is toward the inside of the device. Conversely, as used herein, the term "out" or "outwardly" refers to a location with respect to the device that, during normal use, is toward the outside of the device.

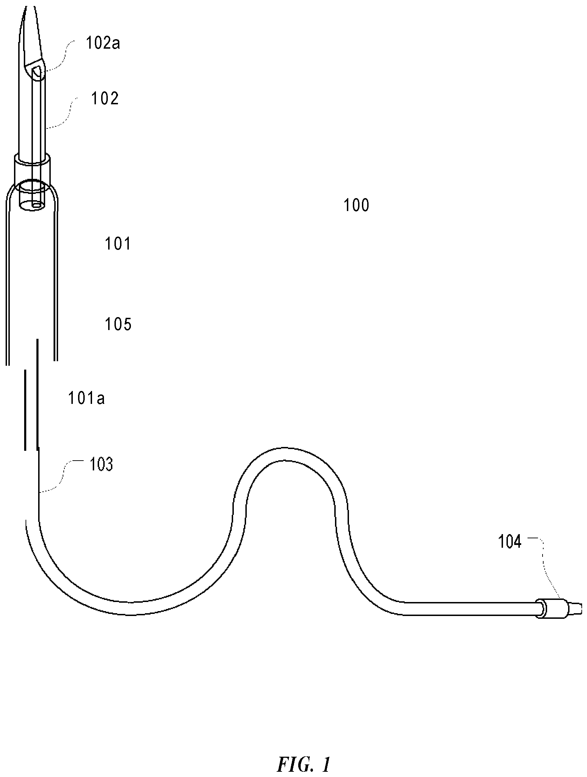

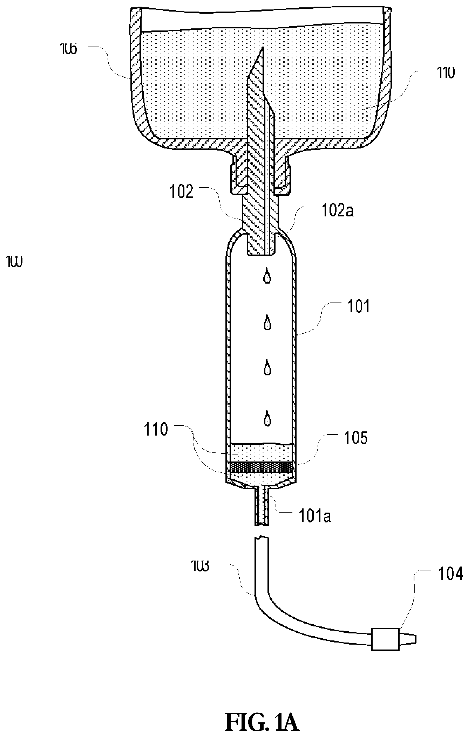

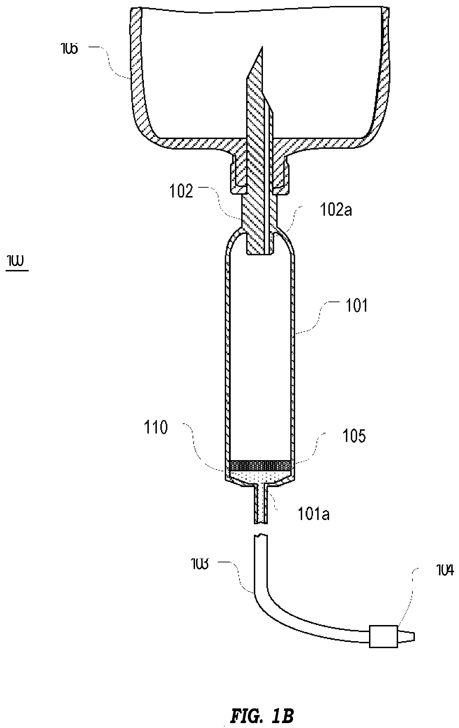

Referring now to Figure 1, a first embodiment of an IV set 100 that includes an air stop membrane is shown. IV set 100 includes a drip chamber 101, a spike 102 for coupling IV set 100 to a fluid bag or other fluid reservoir, tubing 103 for carrying fluid from drip chamber 101 to the patient, and a coupler 104 for coupling tubing 103 to an intravenous device such as a catheter. Typically, spike 102 would be coupled to a fluid bag that is elevated above the patient to allow gravity to pull the fluid through the IV set. However, spike 102 may also be coupled to a fluid bag that employs a pump to distribute the fluid. Spike 102 forms a fluid pathway 102a to allow fluid to flow from a fluid bag into drip chamber 101. Drip chamber 101 includes a distal opening 101a through which fluid flows from drip chamber 101 and into tubing 103. Coupler 104 can typically be a male Luer connector although any suitable connector could be employed.

Although not shown, IV set 100 (and other IV sets described in this specification) can include additional components that are commonly employed on an IV set including a clamp for sealing or constricting tubing 103, a flow controller (other than a clamp) for controlling the flow rate of fluid through tubing 103, auto-level features, zero-dead space connectors, back-check valves, bubble and/or particulate filters, etc.

IV set 100 includes an air stop membrane 105 that is positioned within drip chamber 101. Air stop membrane 105 can be positioned at or near the bottom of drip chamber 101 to ensure that it is positioned below the fluid level during use of the IV set to administer a fluid. Air stop membrane 105 can be formed of a hydrophilic porous material that spans the fluid path in drip chamber 101 to prevent fluid from bypassing the membrane. Air stop membrane 105 can be coupled to the inside surface of drip chamber 101 in any suitable manner including using ultrasonic attachment methods. Directly attaching the membrane to the drip chamber minimizes the number of seals or leak paths and is also the most economically efficient.

Because it is formed of a hydrophilic porous material, air stop membrane 105 will wet quickly once fluid enters into drip chamber 101 thereby causing fluid to flow quickly into tubing 103. As such, the inclusion of air stop membrane 105 does not substantially slow the priming process.

During administration of a fluid, IV set 100 can be employed in a similar manner as prior art IV sets. For example, Figure 1A illustrates a cross-sectional view of IV set 100 when spike 102 has been coupled to a fluid bag 106 for administration of a fluid to a patient. As shown, with spike 102 inserted into fluid bag 106, fluid 110 flows through fluid pathway 102a and into drip chamber 101 where a pool of fluid 110 is formed. Fluid 110 passes through air stop membrane 105, through distal opening 101a, and into tubing 103. During normal operation (i.e., while fluid 110 remains within fluid bag 106), the level of fluid 110 within drip chamber 101 remains substantially constant due to the equilibrium between the rate at which fluid 110 drips into drip chamber 101 and the rate at which fluid 110 exits connector 104 into the patient. As will be further described below, the parameters of air stop membrane 105 can be configured to ensure that the flow rate of fluid 110 through air stop membrane 105 is sufficient for administering a fluid via IV set 100.

Referring now to Figure 1B, IV set 100 is shown once fluid 110 has emptied from fluid bag 106. With fluid bag 106 empty, the level of fluid 110 within drip chamber 101 will continue to decrease from the level shown in Figure 1A. In prior art designs which do not include air stop membrane 105, the fluid would continue to flow out through tubing 103 until the fluid has fully passed into the patient thereby requiring re-priming of the IV set. However, by employing air stop membrane 105, once the level of fluid 110 has reached air stop membrane 105, the flow of fluid 110 will stop as shown in Figure 1B thereby maintaining a fluid column below air stop membrane 105. Air stop membrane 105 can maintain this fluid column until spike 102 can be coupled to a new fluid bag at which point fluid will again flow into drip chamber 101. When coupling to the new fluid bag, there is no need to re-prime the IV set since tubing 103 will already be primed (i.e., full of fluid).

To cause a fluid column to be maintained below air stop membrane 105, air stop membrane 105 can be configured to have a bubble point pressure that is greater than the head of the fluid column or the pressure generated from rapidly decelerating a moving fluid column. Bubble point pressure is a term of art that generally refers to the amount of pressure (or pressure differential) that is required to force air through a membrane. For purposes of this specification, the bubble point pressure can be viewed as the amount of pressure at the top surface of air stop membrane 105 that is required to cause a bubble to pass from the top surface to the bottom surface of air stop membrane 105.

Because air stop membrane 105 is formed of a hydrophilic porous material, fluid will be retained within the pores of the membrane even after the level of fluid 110 has reached air stop membrane 105. Due primarily to capillary action, air above air stop membrane 105 will be blocked from passing through the pores of air stop membrane 105. Since air cannot pass through air stop membrane 105, once fluid 110 reaches the top surface of air stop membrane 105, fluid 110 will cease flowing out of tubing 103 thereby maintaining the fluid column as shown in Figure 1B. The fluid column can be maintained for a substantial amount of time (e.g., until liquid within air stop membrane 105 evaporates, air leaked through tubing, connections, etc., or tubing 103 is disturbed sufficiently to free some of the fluid it holds) thereby allowing a clinician a substantial amount of time to replace fluid bag 106. As a result, IV set 100 can be continuously used to administer multiple fluid bags without re-priming. Given that IV sets are oftentimes used for up to five days, the use of air stop membrane 105 can significantly reduce the clinician's workload.

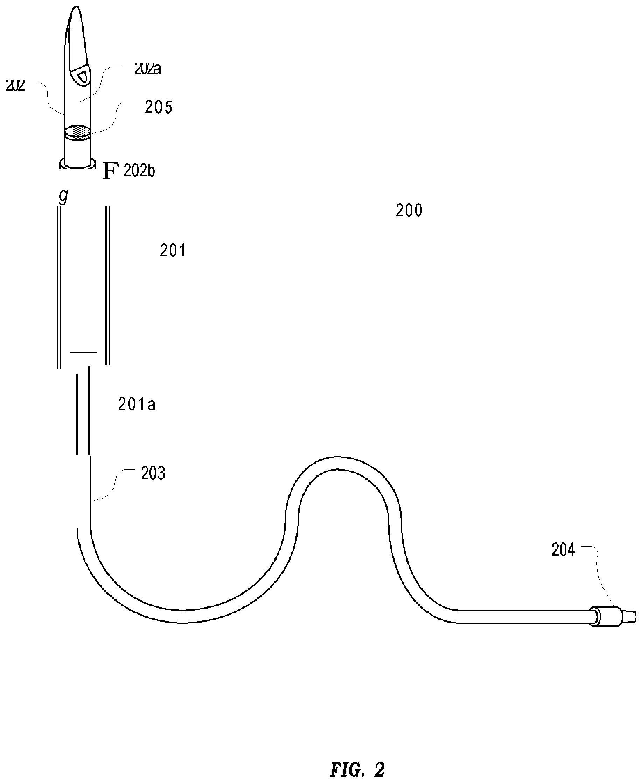

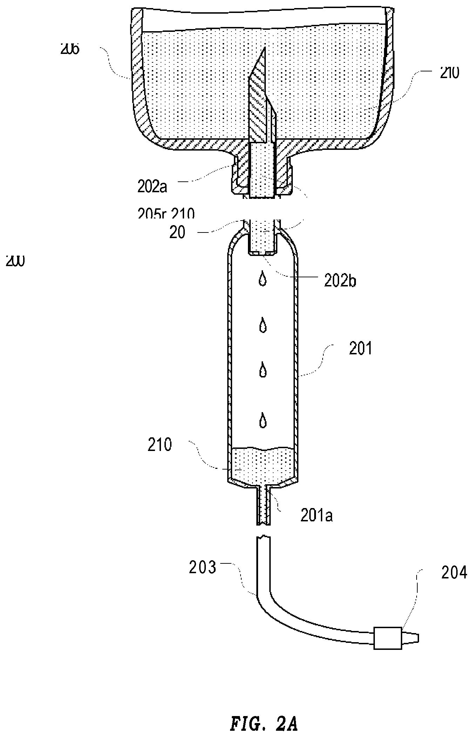

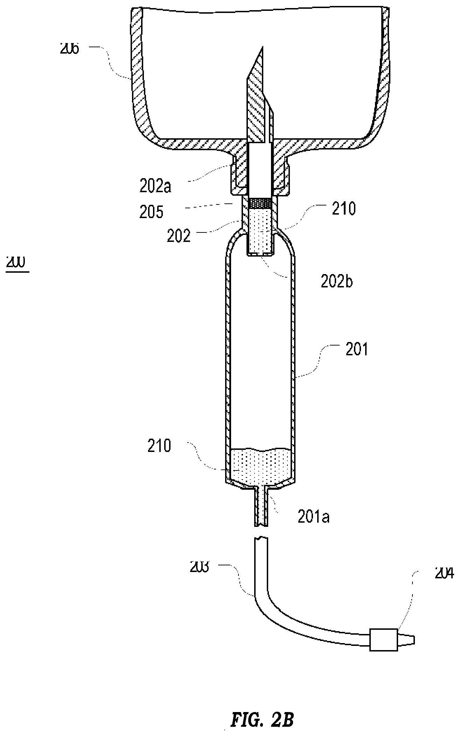

Referring now to Figure 2, a second embodiment of an IV set 200 that includes an air stop membrane is shown. IV set 200 is similar to IV set 100 except that IV set 200 includes an air stop membrane 205 that is positioned in a chamber 202a within spike 202. For purposes of this specification, a spike can be construed as including an air stop membrane if the air stop membrane is positioned in a chamber that is above the drip chamber.

As with IV set 100, IV set 200 includes a drip chamber 202 coupled to spike 202, tubing 203 coupled to drip chamber 202, and a coupler 204 configured to connect tubing 203 to a vascular access device. Spike 202 includes a distal opening 202b through which fluid drips into drip chamber 201. Similarly, drip chamber 201 includes a distal opening 201a through which fluid passes into tubing 203.

Figures 2A and 2B provide cross-sectional views of IV set 200 while spike 202 is coupled to a fluid bag 206. In Figure 2A, fluid bag 206 still contains fluid 210 and therefore fluid 210 is present within chamber 202a both above and below air stop membrane 205, in drip chamber 201, and in tubing 203.

In contrast, in Figure 2B, fluid bag 206 has emptied. As a result, the level of fluid 210 within chamber 202a has decreased until reaching the top surface of air stop membrane 205. At this point, for the reasons described above, air will be blocked from passing through air stop membrane 205, and as a result, fluid 210 will remain within chamber 202a below air stop membrane 205. Because fluid 210 remains within chamber 202a, the pressure within drip chamber 201 will remain substantially constant thereby preventing the flow of fluid 210 through tubing 203. As a result, a fluid column will be maintained within tubing 203 and drip chamber 201. Because air stop membrane 205 maintains a fluid column within tubing 203, fluid bag 206 can be replaced without re-priming IV set 200.

The air stop membrane configuration should take several factors into consideration. The infusion height is the distance from the membrane to the patient's IV administration site. For gravity IV sets the infusion height is typically around 3-4 Ft (0.9-1.2 meters), but can be higher or lower depending on the configuration of the product and the clinical environment. The density of the infusate along with the infusion height will determine the negative pressure generated below the membrane and hence the minimum bubble point for maintaining a fluid column after the fluid column has reached the membrane. The surface tension of the infusate and air change with temperature, typically decreasing as temperature increases. The contact angle between the membrane and infusate surface is different for each type of infusate, so applicable infusates need to be considered. The flow rate also affects membrane performance requirements. Most infusions are at a very low flow rate (<200 mL/hr), so the momentum of the fluid column is negligible; however, for rapid infusions the momentum of the fluid column generates a pressure spike as the fluid column motion is suddenly arrested by the membrane. Rapid infusions are used to administer fluids in trauma situations, or when patients are severely dehydrated and such. These infusion flow rates can be higher than 1.0 L in 10 minutes. This necessitates an increase in the membrane bubble point to withstand the pressure spike. The specific configuration of air stop membrane 105/205 can vary based on the dimensions and intended use of IV set 100/200. In preferred embodiments, the bubble point pressure of air stop membrane 105/205 can be approximately 5.0 psig, equivalent to a static fluid column of up to 3.5 meters, and will support a rapid infusion height of approximately 1.5 meters. A minimum bubble point of approximately 3.0 psi would be marginal to support a static and rapid infusion height of approximately 4 Ft. To obtain a desired bubble point pressure, air stop membrane 105/205 can be configured with a suitable pore size.

Additionally, air stop membrane 105/205 can be configured with a cross-sectional area that is sufficient to provide a desired trans-membrane flow rate. For example, because air stop membrane 105 is positioned within drip chamber 101 which typically has an inside diameter less than 0.75 inches (which is equivalent to an area of 2.85 cm²), air stop membrane 105 can be configured to provide a trans-membrane flow rate of at least 30 mL/min/cm²/bar. With this configuration, air stop membrane 105 will allow fluid 110 to flow through IV set 100 at a rate of approximately 0.5 L/hr which may be sufficient for specific outpatient or non-rapid infusion scenarios. Of course, variations in the area or trans-membrane flow rate can be made as necessary to obtain this rate of 0.5 L/hr. In order to meet a rapid infusion flow rate of 1 L / 10 minutes, or 6 L/hr, with a 1 meter head, a membrane of 0.75 inch diameter (2.85 cm²) would need to have a trans-membrane flow rating of at least 280 mL/min/cm²/bar.

In contrast to what is shown in Figures 2, 2A, and 2B, in some embodiments, spike 202 can be configured to include a portion having a sufficient size to allow chamber 202a to have an inside diameter of approximately 0.75 inches. In such cases, air stop membrane 205 can be configured with the same parameters as stated above to allow for a flow rate of approximately 0.5 L/hr.

In a preferred embodiment, air stop membrane 105/205 can be a polyethersulfone membrane having an effective pore size of approximately 5 µm, a bubble point pressure of approximately 5.0 psig, a trans-membrane flow rate of approximately 2000 mL/min/cm²/bar, and a cross-sectional area of approximately 1.27 cm² (which corresponds to a 0.5 inch diameter drip chamber). With these parameters, air stop membrane 105/205 will allow a flow rate greater than 6.0 L/hr while maintaining a static fluid column (e.g., when tubing 103/203 remains stationary) of 3.5 meters and a dynamic fluid column (e.g., when fluid flowing through tubing 103/203 is decelerating) of at least 1.2 meters.

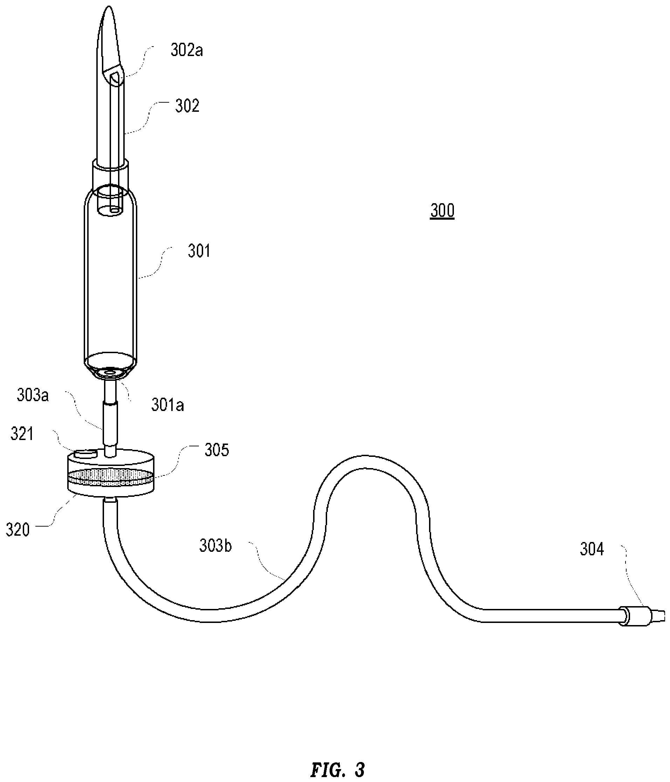

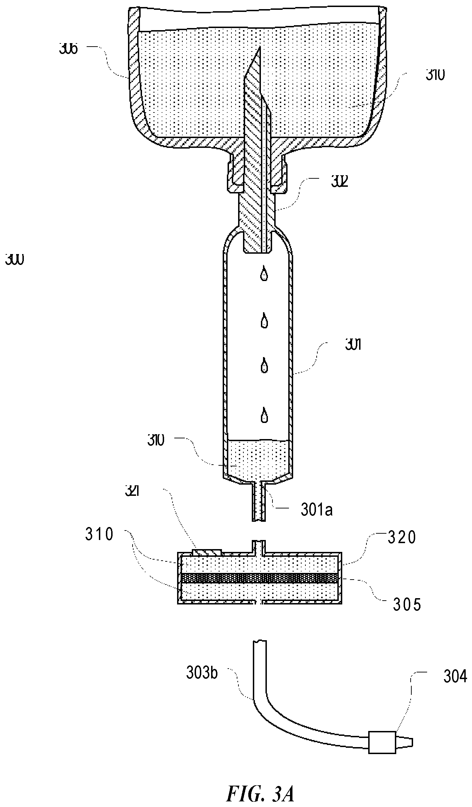

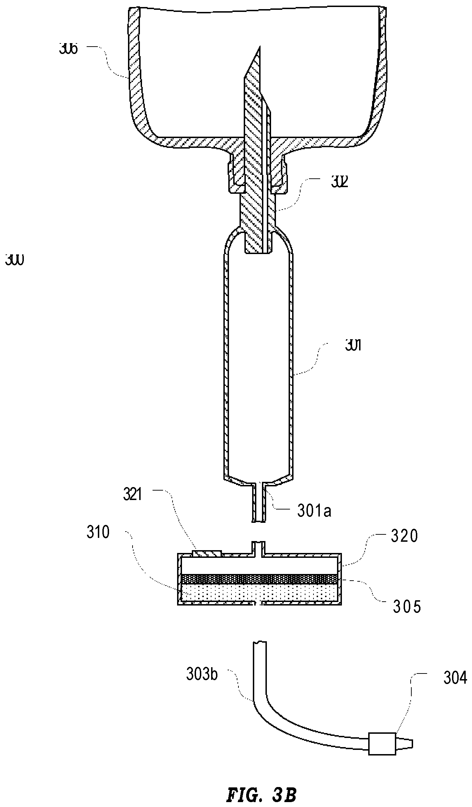

Referring now to Figure 3, a third embodiment of an IV set 300 that includes an air stop membrane is shown. IV set 300 includes a drip chamber 301 and a spike 302 that includes a fluid pathway 302a through which fluid from a fluid bag flows into drip chamber 301. IV set 300 also includes a membrane housing 320 within which an air stop membrane 305 is positioned. Tubing 303a couples drip chamber 301 to membrane housing 320 while tubing 303b extends from a distal opening of membrane housing 320 and includes a coupler 304 for coupling tubing 303b to a vascular access device. Membrane housing 320 also includes an air vent 321 through which air may be vented during priming of IV set 300. Air vent 321 can include a hydrophobic membrane or other filter that allows air but not fluid to pass. Air vent 321 can preferably be configured to allow IV set 300 to be primed in less than 15 seconds. In some embodiments, membrane housing 320 can be configured to couple directly to drip chamber 301 such that tubing 303a is not required.

Figure 3A provides a cross-sectional view of IV set 300 when spike 302 is coupled to a fluid bag 306. As with IV sets 100 and 200, when coupled to fluid bag 306 and primed, a pool of fluid 310 forms within drip chamber 301. In this state, fluid 310 substantially fills tubing 303b, membrane housing 320, and tubing 303a. As long as fluid bag 306 contains fluid 310, this pool of fluid 310 will remain substantially level within drip chamber 301.

In contrast, once fluid bag 306 has emptied, the level of the pool of fluid 310 within drip chamber 301 will decrease until the level has reached the top surface of air stop membrane 305 within membrane housing 320. At this point, for the reasons provided above, air will be blocked from passing through air stop membrane 305, and as a result, fluid 310 will remain within membrane housing 320 below air stop membrane 305. Because fluid 310 remains within membrane housing 320, a fluid column will be maintained within tubing 303b and membrane housing 320. Fluid bag 306 can therefore be replaced without re-priming IV set 300.

One benefit of employing a separate membrane housing 320 is that membrane housing 320 can have a greater size than drip chamber 301 which in turn allows air stop membrane 305 to have a greater cross-sectional area. The greater cross-sectional area allows air stop membrane 305 to provide a greater trans-membrane flow rate without having to increase the pore size, thus reducing the bubble point pressure of the membrane.

On the other hand, another benefit of employing a separate membrane housing 320 is that it allows air stop membrane 305 to be positioned closer to the patient (i.e., closer to the distal end of tubing 303b) so that the required height of the fluid column can be reduced. In other words, membrane housing 320 could be spaced up to 2 feet below drip chamber 301 so that the infusion height is reduced from 3-4 feet to 1-2 Ft (0.3-0.6 m). A bubble point pressure of approximately 1.0 psig would support a static infusion height of 2 feet; the bubble point would need to be greater than this to arrest a rapidly flowing fluid column. The lower bubble point membrane would in turn allow for a higher trans-membrane flow rate thereby minimizing the cross-sectional area required to provide the desired flow rate. Preferably, membrane housing 320 would be spaced no more than 2 feet below drip chamber 301 since the time required to prime IV set 300 is dependent on the IV set internal volume and thus the length of tubing 303a.

In a preferred embodiment, air stop membrane 305 can be a polyethersulfone membrane having an effective pore size of approximately 5 µm, yielding a bubble point pressure of approximately 5.0 psig, a trans-membrane flow rate of approximately 2000 mL/min/cm²/bar, and a cross-sectional area of approximately 2.85 cm² (which corresponds to a 0.75 inch diameter membrane housing). With these parameters, air stop membrane 305 will allow a flow rate greater than 6.0 L/hr.

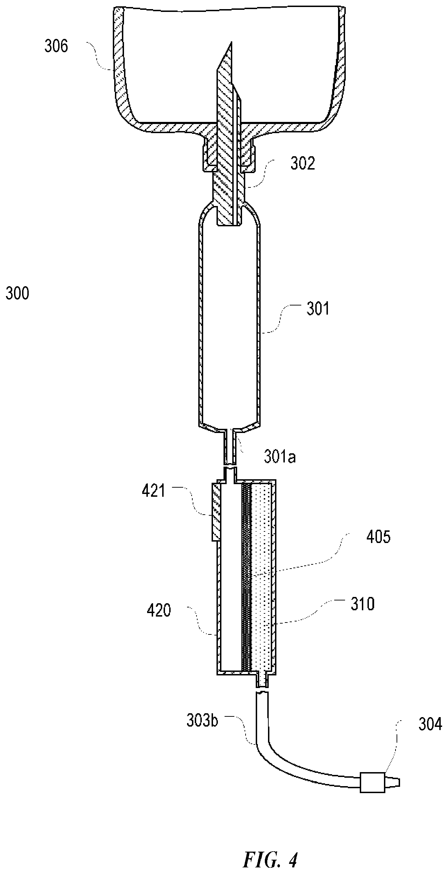

Figure 4 illustrates a variation of IV set 300 in which a membrane housing 420 is used in place of membrane housing 320. Membrane housing 420 differs from membrane housing 320 in that air stop membrane 405 is oriented vertically rather than horizontally. Membrane housing 420 also includes an air vent 421 similar to air vent 321.

Although not shown in the figures, a separate membrane housing 320/420 could be used in an IV set that employs a remote spike. In other words, membrane housing 320/420 could be positioned between the drip chamber and a remote spike. In such embodiments, air stop membrane 305/405 would maintain a fluid column in a similar manner as shown in Figure 2B.

Membrane housing 320/420 can be configured as two-piece components. Membrane 305/405 can be placed between the two pieces and the two pieces can be adhered in any suitable manner (e.g., ultrasonic welding, heat staking, laser welding, etc.) to form a seal around membrane 305/405. Also, when membrane housing 320/420 is formed from two pieces, membrane housing 320/420 can preferably be configured with a round shape so that alignment is not required when coupling the two pieces. However, membrane housing 320/420 can be in any shape including square and rectangular shapes.

In embodiments where IV set 100/200/300 is not configured to auto prime, air stop membrane 105/205/305/405 can be configured to have a wet time that is greater than one second to ensure proper flushing of tubing 103/203/303a/303d (and any connected components) during priming. This ensures that air is not introduced into the tubing below the membrane is the drip chamber is squeezed multiple times during priming. The air stop membrane 105/205/305/405 can be configured to have a wet time that is not more than 60 seconds so as to not interfere with the time required to prime IV set 100/200/300.

In any of the above described embodiments, the air stop membrane can be included in a PVC, DEHP-free, or PVC-free IV set. The air stop membrane may also be included in an IV set that is gravity-fed or pump compatible. The air stop membrane may also be included in an IV set that is used to administer hazardous drugs, or light-sensitive drugs. Any of spikes 102, 202, or 302 can be a macro drip spike that provides 20 drops/mL, a micro drip spike that provides 60 drops/mL, or another drip spike that provides a different drip volume.

In any of the above described embodiments, the air stop membrane can be replaced with another component that provides an air stop function for maintaining a fluid column. For example, a check valve with or without a biasing spring, a ball valve, or a needle and seat design could be employed. With each of these alternatives, the components can be configured to provide a suitable bubble point pressure and flow rate as described above.

The present invention may be embodied in other specific forms without departing from its structures, methods, or other essential characteristics as broadly described herein and claimed hereinafter. The described embodiments are to be considered in all respects only as illustrative, and not restrictive. The scope of the invention is, therefore, indicated by the appended claims, rather than by the foregoing description. All changes that come within the meaning and range of equivalency of the claims are to be embraced within their scope.

### Further aspects of the invention:

1. An IV set comprising:
   a one piece drip chamber;
   a spike for fluidly coupling the drip chamber to a fluid bag;
   tubing for coupling the drip chamber to a vascular access device; and
   an air stop membrane directly attached to the drip chamber which maintains a fluid column below the membrane after a fluid level within the IV set reaches the membrane.
2. The IV set of 1 wherein the air stop membrane is a hydrophilic membrane.
3. The IV set of 3 wherein the air stop membrane is located in the drip chamber.
4. The IV set of 1, wherein the air stop membrane is adhered to a bottom surface of the drip chamber overtop a distal opening of the drip chamber.
5. The IV set of 3 wherein the membrane bubble point pressure is greater than 3 psi.
6. The IV set of 5 wherein the cross-sectional flow area is less than 2.85 cm2
7. The IV set of 1 wherein the trans-membrane flow is greater than 30 mL/min/cm2/bar.
8. The IV set of 6 wherein the trans-membrane flow is greater than 1150 mL/min/cm2/bar.
9. An IV set comprising;
   a drip chamber
   a spike for fluidly coupling the drip chamber to a fluid bag;
   tubing for coupling the drip chamber to a vascular access device; and
   an air stop membrane positioned within the spike or between the spike and the drip chamber wherein the membrane maintains a fluid column below the membrane after a fluid level within the IV set reaches the membrane.
10. The IV sect of 9 wherein the membrane bubble point pressure is greater than 3 psi.
11. The IV set of 9 wherein the membrane bubble point pressure is approximately 5 psi.
12. The IV set of 9 wherein the cross-sectional flow area is less than 2.85 cm2
13. The IV set of 9 wherein the trans-membrane flow is greater than 30 mL/min/cm2/bar.
14. An IV set comprising:
   a drip chamber;
   a spike for fluidly coupling the drip chamber to a fluid bag;
   tubing for coupling the drip chamber to a vascular access device; and
   an air stop membrane attached to the IV set which maintains a fluid column below the membrane after a fluid level within the IV set reaches the membrane;
   further comprising a membrane housing, the membrane being positioned within the membrane housing.
15. The IV sect of 14 wherein the wherein the air stop membrane has a bubble point pressure greater than 1 psi.
16. The IV set of 14 wherein the cross-sectional flow area is less than 60 cm2
17. The IV set of 14 wherein the trans-membrane flow is greater than 30 mL/min/cm2/bar.
18. The IV set of 14 wherein the trans-membrane flow is approximately 2000 mL/min/cm2/bar.
19. The IV set of 14, wherein the membrane housing is positioned downstream and separate from the drip chamber or between the drip chamber and remote spike.

## Claims

1. An IV set comprising:
a drip chamber (301);
a spike (302) for fluidly coupling the drip chamber to a fluid bag;
tubing (303a) for coupling the drip chamber to a vascular access device; and
an air stop membrane (305) attached to the IV set which maintains a fluid column below the membrane after a fluid level within the IV set reaches the membrane;
further comprising a membrane housing (320), the membrane being positioned within the membrane housing.

2. The IV sect of claim 1 wherein the wherein the air stop membrane (305) has a bubble point pressure greater than 1 psi.

3. The IV set of claim 1 wherein the cross-sectional flow area is less than 60 cm2.

4. The IV set of claim 1 wherein the trans-membrane flow is greater than 30 mL/min/cm2/bar.

5. The IV set of claim 1 wherein the trans-membrane flow is approximately 2000 mL/min/cm2/bar.

6. The IV set of claim 1, wherein the membrane housing (320) is positioned downstream and separate from the drip chamber (301) or between the drip chamber and remote spike .

7. The IV set of claim 1, wherein the air stop membrane (305) is vertically-oriented.
